# EUROPEAN PATENT SPECIFICATION

(11) **EP 4 248 890 B1**
(45) Date of publication and mention of the grant of the patent: **21.05.2025**
(21) Application number: 23162811.6
(22) Date of filing: 20.03.2023
(51) Int. Cl.: A61B 17/80

(54) **VETERINARY DISTAL HUMERUS FRACTURE PLATES**
DISTALE HUMERUS-FRAKTURPLATTEN FÜR DIE VETERINÄRMEDIZIN
PLAQUES VÉTÉRINAIRES DISTALES DE FRACTURE DE L'HUMÉRUS

(30) Priority: 23.03.2022 US 202263322969 P
(43) Date of publication of application: 27.09.2023
(73) Proprietor: Arthrex Inc, Naples, FL 34108-1945 (US)
(72) Inventor: KARNES, G. Joshua, Estero, FL, 33928 (US); CROWLEY, Casey, Arvada, CO, 80004 (US); HOUBRE, Matthew, Fort Myers, FL, 33912 (US); MURPHY, Sean, Boise, ID, 83702 (US); WALLS, Charles, Clayton, CA, 94517 (US); HOLSWORTH, Ian, Ventura, CA, 93003 (US)
(74) Representative: Lohr, Jöstingmeier & Partner

(56) References cited:
- US-A1- 2005 240 187
- US-A1- 2015 127 011
- US-A1- 2016 310 184
- US-A1- 2020 163 703
- US-A1- 2021 361 331

## Description

### BACKGROUND

Canines, felines, or other animals may suffer a bone fracture as a result of a traumatic impact. Bone fractures are typically repaired using a combination of a bone plate and fixation screws. Veterinary bone plates and fixation systems thereof are described herein.

US 2020/0163703 A1 discloses a bone plate with the features of the preamble of claim 1.

Further bone plates of the type claimed are known from US 2005/240187 A1,

US 2015/127011 A1, US 2016/310184 A1 and US 2021/361331 A1.

### SUMMARY

The present invention is defined in claim 1 while preferred embodiments are set forth in the dependent claims.

This disclosure is directed to bone plates for repairing distal humerus fractures in animals, such as canines.

According to the invention, a bone plate designed for veterinary use includes a shaft portion, a head portion, a bone contacting surface, and an outer surface opposed to the bone contacting surface and an elliptical hole is formed through the head portion and is configured for receiving a condyle of a distal humerus of an animal.

According to an embodiment of the invention, the shaft portion includes a first width, the head portion includes a second width that is larger than the first width, and the bone contacting surface is configured to conform to a contour of a distal humerus of an animal.

The head portion is bounded by a peripheral edge that connects the head portion to a first side and a second side of the shaft portion. The peripheral edge includes a curved expanded section that connects to the first side of the shaft portion, and an outcropped section that connects to the second side of the shaft portion.

### BRIEF DESCRIPTION OF THE DRAWINGS

Figure 1 illustrates select portions of a canine musculoskeletal system. A humerus of the canine includes a fracture.
Figures 2(a), 2(b), 2(c), and 2(d) illustrate various types of humeral fracture patterns.
Figure 3 is a top view of a bone plate for repairing a distal humerus fracture.
Figure 4 is a bottom view of the bone plate of Figure 3.
Figure 5 is a side view of the bone plate of Figure 3.
Figure 6 illustrates the bone plate of Figures 3, 4, and 5 positioned relative to a distal humerus of an animal.
Figure 7 is a cross-sectional view through a head portion of the bone plate of Figure 3.
Figures 8(a), 8(b), and 8(c) illustrate various views of a bone plate having a left orientation.
Figure 9 is a top view of another exemplary bone plate for repairing a distal humerus fracture.
Figure 10 is a bottom view of the bone plate of Figure 9.
Figure 11 is a side view of the bone plate of Figure 9.
Figure 12 illustrates the bone plate of Figures 9, 10, and 11 positioned relative to a canine distal humerus.
Figure 13 is a cross-sectional view through a head portion of the bone plate of Figure 9.
Figures 14(a), 14(b), and 14(c) illustrate various views of another exemplary bone plate having a left orientation.

### DETAILED DESCRIPTION

This disclosure is directed to bone plates designed for veterinary use. For example, the bone plates could be utilized for repairing distal humerus fractures in canines or felines. These and other features of this disclosure are described in further detail below.

According to the invention, a bone plate for veterinary use includes a shaft portion, a head portion, a bone contacting surface, and an outer surface opposed to the bone contacting surface. An elliptical hole is formed through the head portion and is configured for receiving a condyle of a distal humerus of an animal.

In a further embodiment, a bone plate includes a bone contacting surface having a curvature adapted for conforming to a contour of a canine distal humerus.

In a further embodiment, a relief is formed in a bone contacting surface of a head portion of a bone plate.

In a further embodiment, a shaft portion of a bone plate includes a first length that is greater than a second length of a head portion of the bone plate.

In a further embodiment, a head portion of a bone plate includes a first width that is greater than a second width of a shaft portion of the bone plate.

In a further embodiment, a plurality of openings and at least one K-wire hole are formed through a shaft portion of a bone plate.

In a further embodiment, a plurality of openings and at least one K-wire hole are formed through a head portion of a bone plate.

In a further embodiment, a head portion of a bone plate is bounded by a peripheral edge that connects the head portion to a first side and a second side of a shaft portion of the bone plate.

In a further embodiment, a peripheral edge of a head portion of a bone plate includes a curved expanded section that connects to a first side of a shaft portion of the bone plate and an outcropped section that connects to a second side of the shaft portion.

In a further embodiment, a first opening is located proximate a curved expanded section of a head portion of a bone plate, and a second opening is located proximate an outcropped section of the head portion.

In a further embodiment, a first opening located proximate a curved expanded section of a head portion of a bone plate includes a larger diameter than a second opening located proximate an outcropped section of the head portion.

In a further embodiment, a peripheral edge of a head portion of a bone plate includes a curved expanded section that connects to a first side of a shaft portion of the bone plate and a straight edge that connects to a second side of the shaft portion.

In a further embodiment, a first opening is located proximate a curved expanded section of a head portion of a bone plate, and a second opening, a third opening, and a fourth opening are located proximate to a straight edge of a head portion.

In a further embodiment, an elliptical hole of a bone plate is positioned axially between first, second, and third openings of a head portion of the bone plate.

In a further embodiment, a head portion of a bone plate is angled at an inclined angle relative to a shaft portion of the bone plate.

In a further embodiment, the shaft portion includes a first width, a head portion includes a second width that is larger than the first width, and the bone contacting surface is configured to conform to a contour of a distal humerus of an animal.

The head portion is bounded by a peripheral edge that connects the head portion to a first side and a second side of the shaft portion. The peripheral edge includes a curved expanded section that connects to the first side of the shaft portion and an outcropped section that connects to the second side of the shaft portion

In a further embodiment, an elliptical hole is formed through a head portion of a bone plate and is configured for receiving a condyle of a canine distal humerus.

In a further embodiment, an elliptical hole is positioned axially between a first opening located proximate a curved expanded section of a head portion of a bone plate and a third opening and a fourth opening formed proximate a straight section that connects to an outcropped section of the head portion.

In a further embodiment, a fourth opening is located proximate an outcropped section of a head portion of a bone plate.

In a further embodiment, a shaft portion of a bone plate includes a proximal tip section that is angled relative to a distal straight section of the shaft portion.

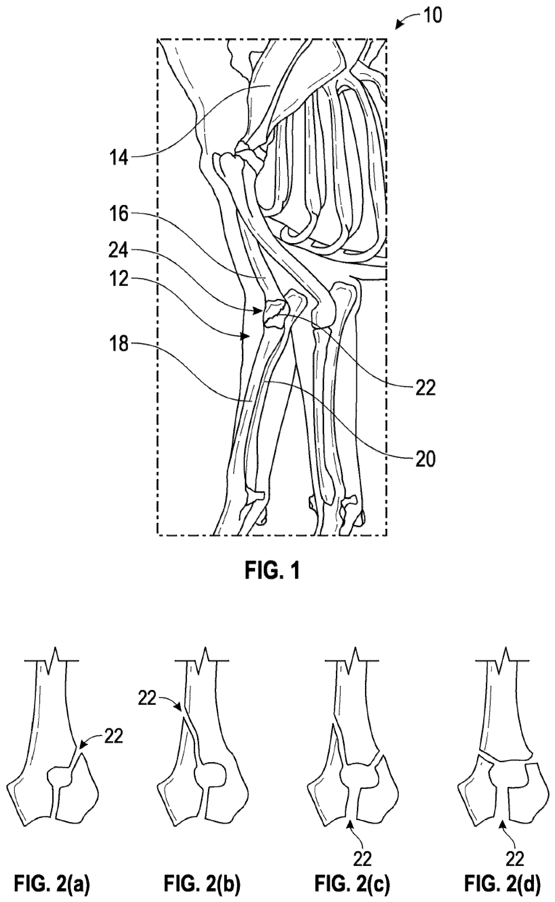

Figure 1 schematically illustrates select portions of a musculoskeletal system 10 of an animal. In an embodiment, the musculoskeletal system 10 is that of a canine. However, the teachings of this disclosure may be applicable for other veterinary uses, such as for felines, for example.

Forelimb portions 12 of the musculoskeletal system 10 are specifically shown in Figure 1. Each forelimb portion 12 includes, among other bones, a scapula 14, a humerus 16 positioned distally of the scapula 14, and a radius 18 and an ulna 20 positioned distally of the humerus 16. The humerus 16 interfaces with the scapula 14 to establish a shoulder joint of the animal, and the radius 18 and the ulna 20 interface with the humerus 16 to establish an elbow joint of the animal.

As schematically illustrated, a fracture 22 may occur in a distal section 24 of the humerus 16, such as in response to a traumatic impact. The distal section 24 may be simply referred as the distal humerus. The fracture 22 may present as a lateral fracture pattern (see Figure 2(a)), a medial fracture pattern (see Figure 2(b)), a "Y" type fracture pattern (see Figure 2(c)), or a "T" type fracture pattern (see Figure 2(d)). Whatever pattern exhibited by the fracture 22, the fracture 22 typically must be repaired in order to alleviate pain and facilitate proper bone healing. The fracture 22 may be repaired using a bone plate. This disclosure is therefore directed to bone plate designs that are suitable for repairing distal humerus fractures of animals (e.g., non-humans such as canines and felines).

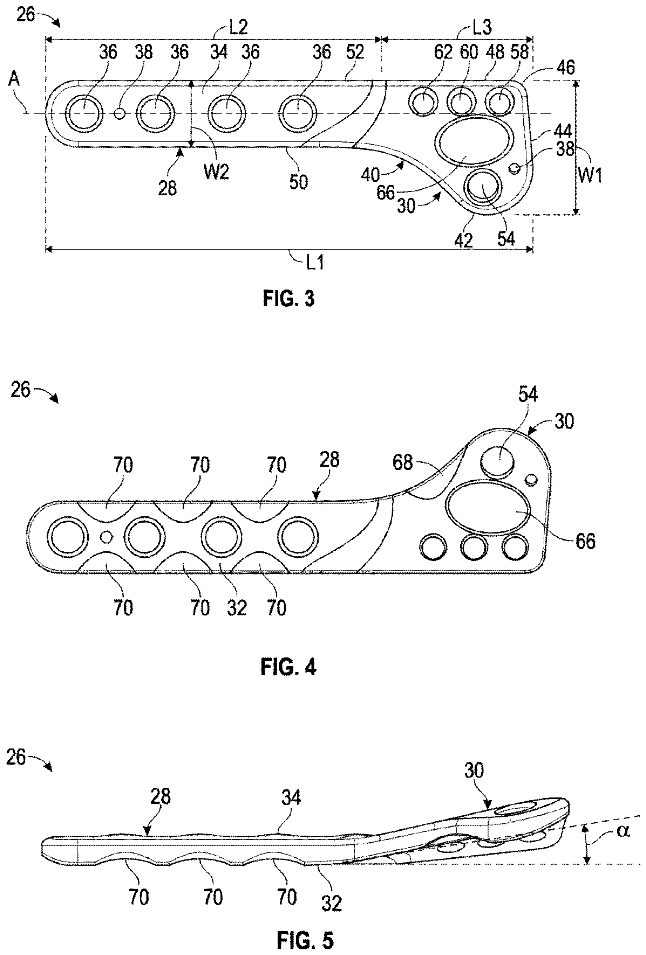

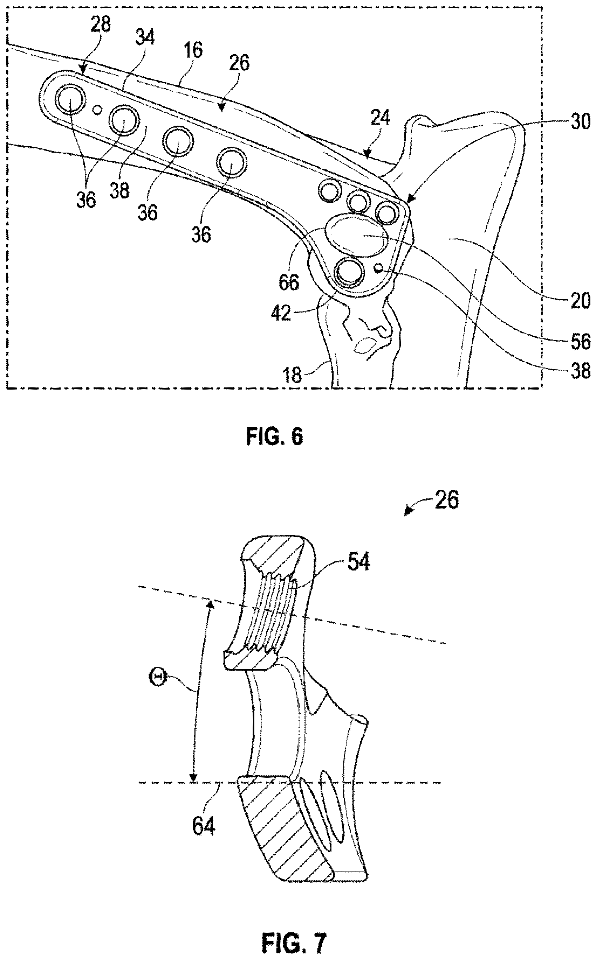

Figures 3-7 illustrate an exemplary bone plate 26 for repairing a bone defect, such as a fracture 22 of a distal section 24 of a humerus 16 of an animal, such as a canine or feline, for example. The bone plate 26 is shown alone in Figures 3, 4, and 5, and the bone plate 26 is shown positioned relative to the distal section 24 of the humerus 16 in Figure 6.

The bone plate 26 may extend along a longitudinal axis A between a shaft portion 28, located proximally when the bone plate 26 is implanted, and a head portion 30, located distally when the bone plate 26 is implanted (see, e.g., Figure 6). The shaft portion 28 and the head portion 30 establish a single-piece bone plate structure.

The bone plate 26 includes an overall length L1. The overall length L1 may be specifically sized for use relative the distal section 24 of the humerus 16 of a canine or a feline, for example.

The shaft portion 28 may include a length L2, and the head portion 30 may include a length L3. In an embodiment, the length L2 is larger than the length L3.

The head portion 30 may be broader than the shaft portion 28. For example, the head portion 30 may include a maximum width W1 that is larger than a maximum width W2 of the shaft portion 28.

The head portion 30 may extend at an inclined angle relative to the shaft portion 28. In an embodiment, the head portion 30 is angled at an inclined angle α of between about 10 degrees and about 15 degrees relative to the shaft portion 28 (see Figure 5). In this disclosure, the term "about" means that the expressed quantities or ranges need not be exact but may be approximated and/or larger or smaller, reflecting acceptable tolerances, conversion factors, measurement error, etc.

The bone plate 26 may further include a bone contacting surface 32 and an outer surface 34 on an opposite side of the bone plate 26 from the bone contacting surface 32. The bone contacting surface 32 may include a slightly concave curvature for conforming to the contour of the distal section of 24 of the humerus 16.

The shaft portion 28 may include a plurality of openings 36 configured for receiving fixation devices (e.g. screws, etc., not shown in Figures 3-6) for fixating the bone plate 26 to the distal section 24 of the humerus 16. The openings 36 extend completely through the shaft portion 28 and therefore open through both the bone contacting surface 32 and the outer surface 34. In an embodiment, the shaft portion 28 includes four openings 36 that are equally sized and aligned relative to one another along the longitudinal axis A. However, other configurations are also contemplated, and thus the total number of openings 36 and their specific arrangement within the shaft portion 28 are not intended to limit this disclosure.

The shaft portion 28 may additionally include one or more K-wire holes 38 configured for receiving a K-wire (not shown) in order to temporarily secure the bone plate 26 to the distal section 24 of the humerus 16 prior to achieving final fixation via fixation screws. The K-wire holes 38 extend completely through the shaft portion 28 and therefore open through both the bone contacting surface 32 and the outer surface 34. The total number of K-wire holes 38 provided in the shaft portion 28 is not intended to limit this disclosure.

The head portion 30 may be bounded by a peripheral edge 40. The peripheral edge 40 may connect to and extend outwardly of a first side 50 of the shaft portion 28 to establish a curved expanded section 42 of the head portion 30. From the curved expanded section 42, the peripheral edge 40 may continue linearly to establish a straight distal section 44 of the head portion 30. The peripheral edge 40 may extend from the straight distal section 44 about a curved edge 46 and then linearly along a straight section 48 that is positioned on an opposite side of the head portion 30 from the curved expanded section 42. The straight section 48 may connect to a second side 52 of the shaft portion 28.

A first opening 54 may be formed through the head portion 30 at a location that is proximate to the curved expanded section 42. The first opening 54 may extend completely through the head portion 30 and therefore opens through both the bone contacting surface 32 and the outer surface 34. The first opening 54 may be configured to receive a transcondylar screw that allows for the capture of both condyles 56 of the distal section 24 of the humerus 16 with a single screw.

A second opening 58, a third opening 60, and a fourth opening 62 may be formed through the head portion 30 at a location that is proximate to the straight section 48. The second opening 58, the third opening 60, and the fourth opening 62 may extend completely through the head portion 30 and therefore open through both the bone contacting surface 32 and the outer surface 34. Each of the second opening 58, the third opening 60, and the fourth opening 62 is configured for receiving a fixation device (e.g. a screw, etc., not shown in Figures 3-6) for fixating the bone plate 26 to the distal section 24 of the humerus 16.

In an embodiment, the second opening 58, the third opening 60, and the fourth opening 62 are equally sized openings. In another embodiment, the first opening 54 is larger in diameter compared to any of the second opening 58, the third opening 60, or the fourth opening 62.

Each of the first opening 54, the second opening 58, the third opening 60, and the fourth opening 62 may be an angled opening that positions a fixation screw received therein within a safe corridor across one of the condyles 56 of the distal section 24 of the humerus 16. In an embodiment, the openings 54, 58, 60, 62 extend at an angle Θ relative to a transverse axis 64 that extends vertically through the head portion 30 (see Figure 7). The transverse axis 64 is perpendicular to the longitudinal axis A of the bone plate 26. In an embodiment, the angle Θ is about 10 degrees. However, other angles may also be suitable.

The head portion 30 may additionally include an elliptical hole 66. The elliptical hole 66 may be sized and shaped for accommodating at least a portion of one of the condyles 56 of the distal section 24 of the humerus 16, thereby providing a better anatomic fit. The elliptical hole 66 may extend completely through the head portion 30 and therefore opens through both the bone contacting surface 32 and the outer surface 34. In an embodiment, the elliptical hole 66 is positioned axially between the first opening 54 and the second, third, and fourth openings 58, 60, 62.

The head portion 30 may additionally include one or more K-wire holes 38 configured for receiving a K-wire (not shown) in order to temporarily secure the bone plate 26 to the distal section 24 of the humerus 16 prior to achieving final fixation via fixation screws. The K-wire holes 38 extend completely through the head portion 30 and therefore open through both the bone contacting surface 32 and the outer surface 34. The total number of K-wire holes 38 provided in the head portion 30 is not intended to limit this disclosure.

A relief 68 (see Figure 4) may be formed in head portion 30 on the bone contacting surface 32 of the bone plate 26. The relief 68 may be disposed slightly proximally of the elliptical hole 66 and the first opening 54. The relief 68 may be sized and shaped to provide clearance over a soft tissue anatomy of the distal section 24 of the humerus 16.

A plurality of scalloped portions 70 (see Figures 4 and 5) may be formed in the shaft portion 28 on the bone contacting surface 32 of the bone plate 26. The scalloped portions 70 are configured to minimize bone contact at certain locations of the bone plate 26, thereby creating a constant stiffness across the bone plate 26 when implanted.

The longitudinal axis A may bisect the shaft portion 28 into two equal sections such that the shaft portion 28 is substantially symmetrical about the longitudinal axis A. The head portion 30 may be asymmetrically disposed about the longitudinal axis A. In an embodiment, the longitudinal axis A may intersect through an edge of each of the second, third, and fourth openings 58, 60, and 62 of the head portion 30 (see Figure 3).

The bone plate 26 may be made from any biocompatible material or combination of biocompatible materials. Exemplary materials that may be suitable for manufacturing the bone plate 26 include, but are not limited to, titanium, titanium alloys, stainless steel, thermoplastic materials, etc.

The various openings 36, 58, 60, and 62 of the bone plate 26 may be configured to receive fixation screws of any size (e.g., 2.0 mm, 2.4 mm, etc.), and some openings could be sized differently than the other openings. Thus, the specific sizes of the openings of the bone plate 26 are not intended to limit this disclosure.

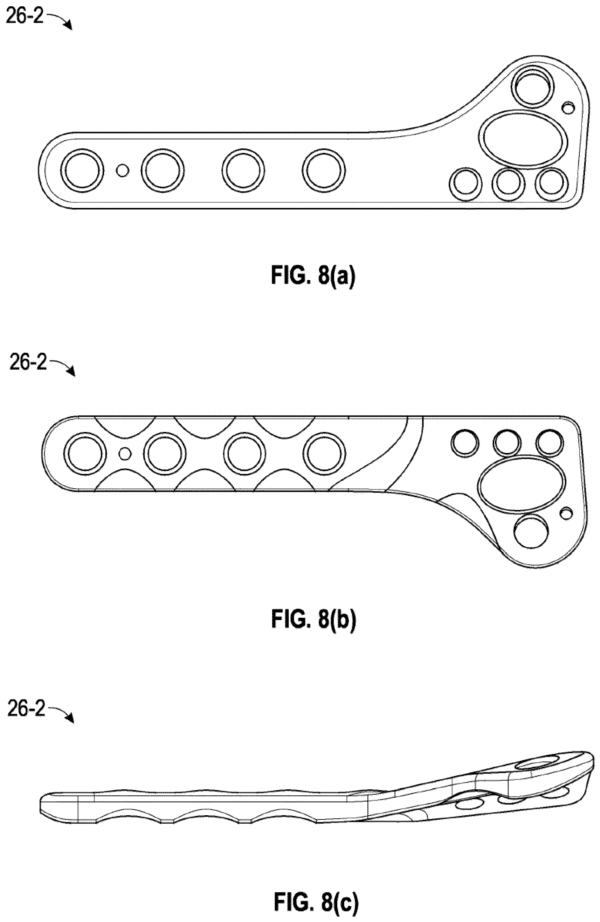

In the above embodiment, the bone plate 26 is shown and described as having a right orientation. However, the bone plate 26 could also be provided in a left orientation, with such a bone plate being the mirror image of the right orientated bone plates described above. Figures 8(a), 8(b), and 8(c) illustrate an exemplary bone plate 26-2 having a left orientation.

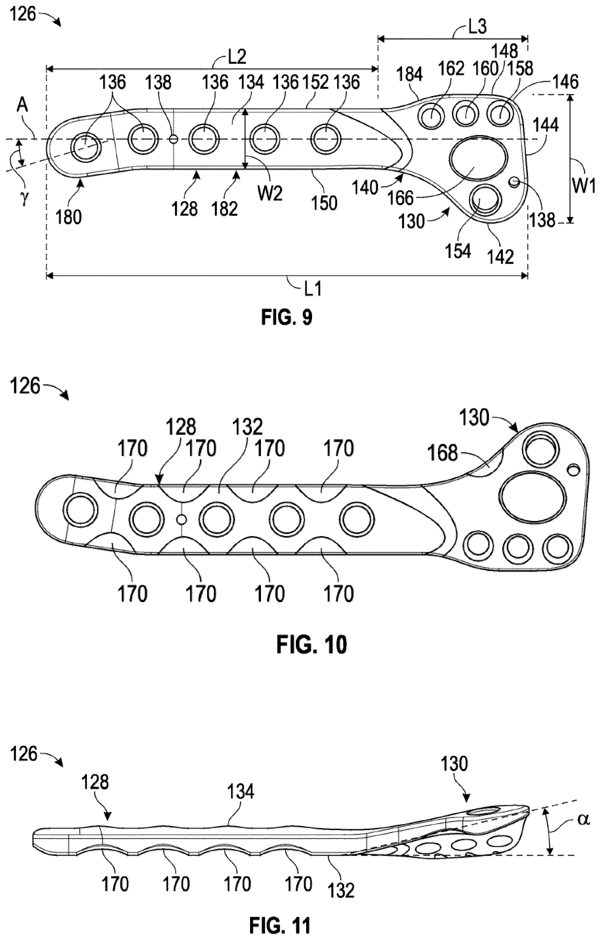

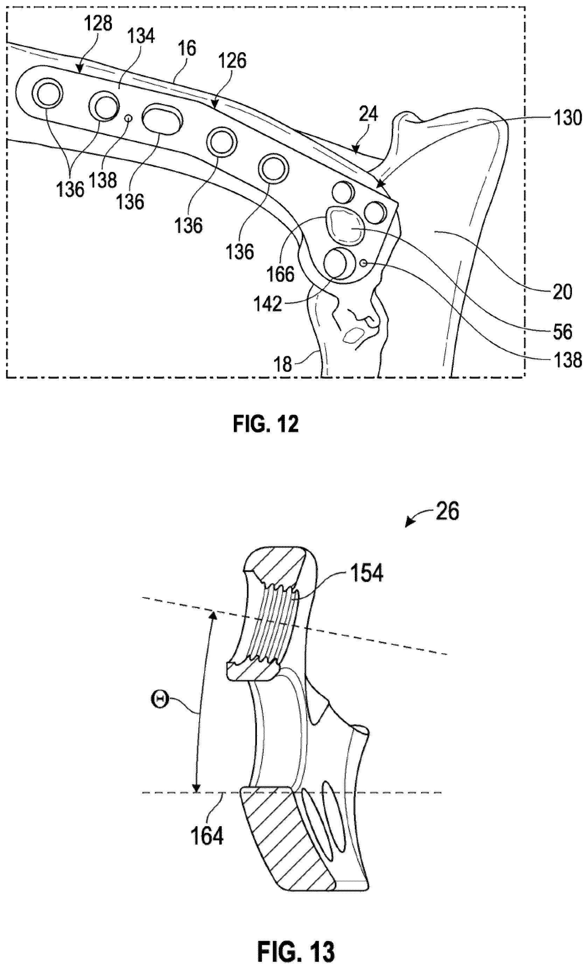

Figures 9-13 illustrate another exemplary bone plate 126 for repairing distal humerus fractures of an animal, such as a canine or a feline, for example. As will become more apparent from the description below, the bone plate 126 is similar to the bone plate 26 of Figure 3-7 but includes some additional/modified features. The bone plate 126 is shown alone in Figures 9, 10, and 11, and the bone plate 126 is shown positioned relative to a distal section 24 of a humerus 16 in Figure 12.

The bone plate 126 may extend along a longitudinal axis A between a shaft portion 128, located proximally when the bone plate 126 is implanted, and a head portion 130, located distally when the bone plate 126 is implanted (see, e.g., Figure 12). The shaft portion 128 and the head portion 130 establish a single-piece bone plate structure.

The bone plate 126 includes an overall length L1. The overall length L1 may be specifically sized for use relative the distal section 24 of the humerus 16 of a canine or a feline, for example.

The shaft portion 128 may include a length L2, and the head portion 130 may include a length L3. In an embodiment, the length L2 is larger than the length L3.

The head portion 130 may be broader than the shaft portion 128. For example, the head portion 130 may include a maximum width W1 that is larger than a maximum width W2 of the shaft portion 128.

The head portion 130 may extend at an inclined angle relative to the shaft portion 128 (see Figure 11). In an embodiment, the head portion 130 is angled at an inclined angle α of between about 10 degrees and about 15 degrees relative to the shaft portion 128. In another embodiment, the inclined angle α is about 13 degrees for allowing the bone plate 126 to better accommodate the animal's anatomy.

The bone plate 126 may further include a bone contacting surface 132 and an outer surface 134 on an opposite side of the bone plate 126 from the bone contacting surface 132. The bone contacting surface 132 may include a slightly concave curvature for conforming to the contour of the distal section of 24 of the humerus 16.

The shaft portion 128 may include a plurality of openings 136 configured for receiving fixation devices (e.g. screws, etc., not shown in Figures 9-12) for fixating the bone plate 126 to the distal section 24 of the humerus 16. The openings 136 extend completely through the shaft portion 128 and therefore open through both the bone contacting surface 132 and the outer surface 134. In an embodiment, the shaft portion 128 includes five openings 136 that are equally sized and spaced along the longitudinal axis A. However, other configurations are also contemplated, and thus the total number of openings 136 and their specific arrangement within the shaft portion 128 are not intended to limit this disclosure.

The shaft portion 128 may additionally include one or more K-wire holes 138 configured for receiving a K-wire (not shown) in order to temporarily secure the bone plate 126 to the distal section 24 of the humerus 16 prior to achieving final fixation via fixation screws. The K-wire holes 138 extend completely through the shaft portion 128 and therefore open through both the bone contacting surface 132 and the outer surface 134. The total number of K-wire holes 138 provided in the shaft portion 128 is not intended to limit this disclosure.

The shaft portion 128 may include a proximal tip section 180 and a distal straight section 182. The proximal tip section 180 may be angled relative to the distal straight section 182 to establish a slight kickout within the shaft portion 128 for providing a more optimized screw placement. The proximal tip section 180 may extend at an angle γ relative to the distal straight section 182. In an embodiment, the angle γ is between about 3 degrees and about 18 degrees. In another embodiment, the angle γ is about 8 degrees. However, the angle γ may be optimized for suitability relative to any type of animal.

A proximal most opening of the openings 136 may be formed through the proximal tip section 180, and the remaining openings 136 may be formed through the distal straight section 182. However, other configurations are alternatively possible.

The head portion 130 may be bounded by a peripheral edge 140. The peripheral edge 140 may connect to and extend outwardly of a first side 150 of the shaft portion 128 to establish a curved expanded section 142 of the head portion 130. From the curved expanded section 142, the peripheral edge 140 may continue linearly to establish a straight distal section 144 of the head portion 130. The peripheral edge 140 may extend from the straight distal section 144 about a curved edge 146 and then linearly along a straight section 148 that is positioned on an opposite side of the head portion 130 from the curved expanded section 142.

An outcropped section 184 of the peripheral edge 140 may be positioned between the straight section 148 and a second side 152 of the shaft portion 128. The outcropped section 184 provides increased plate material in the head portion 130 for maximizing and optimizing bone purchase.

A first opening 154 may be formed through the head portion 130 at a location that is proximate to the curved expanded section 142. The first opening 154 may extend completely through the head portion 130 and therefore opens through both the bone contacting surface 132 and the outer surface 134. The first opening 154 may be configured to receive a transcondylar screw that allows for the capture of both condyles 56 of the distal section 24 of the humerus 16 with a single screw.

A second opening 158 and a third opening 160 may be formed through the head portion 130 at a location that is proximate to the straight section 148. The second opening 158 and the third opening 160 may extend completely through the head portion 130 and therefore open through both the bone contacting surface 132 and the outer surface 134.

A fourth opening 162 may be formed through the head portion 130 at a location that is proximate to the outcropped section 184. In an embodiment, the fourth opening 162 is the proximal most opening formed in the head portion 130. The fourth opening 162 may extend completely through the head portion 130 and therefore opens through both the bone contacting surface 132 and the outer surface 134. Each of the second opening 158, the third opening 160, and the fourth opening 162 is configured for receiving a fixation device (e.g. a screw, etc., not shown in Figures 9-12) for fixating the bone plate 26 to the distal section 24 of the humerus 16.

In an embodiment, the second opening 158, the third opening 160, and the fourth opening 162 are equally sized openings. In another embodiment, the first opening 154 is larger in diameter compared to any of the second opening 158, the third opening 160, or the fourth opening 162.

Each of the first opening 154, the second opening 158, the third opening 160, and the fourth opening 162 may be an angled opening that is configured to position a fixation screw received therein within a safe corridor across one of the condyles 56 of the distal section 24 of the humerus 16. In an embodiment, the openings 154, 158, 160, 162 extend at an angle Θ relative to a transverse axis 164 that extends vertically through the head portion 130 (see Figure 13). The transverse axis 164 is perpendicular to the longitudinal axis A of the bone plate 126. In an embodiment, the angle Θ is about 10 degrees. However, other angles may also be suitable.

The head portion 130 may additionally include an elliptical hole 166. The elliptical hole 166 may be sized and shaped for accommodating at least a portion of one of the condyles 56 of the distal section 24 of the humerus 16, thereby providing a better anatomic fit. The elliptical hole 166 may extend completely through the head portion 130 and therefore opens through both the bone contacting surface 132 and the outer surface 134. In an embodiment, the elliptical hole 166 is positioned axially between the first opening 154 and the second and third openings 158, 160.

The head portion 130 may additionally include one or more K-wire holes 138 configured for receiving a K-wire (not shown) in order to temporarily secure the bone plate 126 to the distal section 24 of the humerus 16 prior to achieving final fixation via fixation screws. The K-wire holes 138 extend completely through the head portion 130 and therefore open through both the bone contacting surface 132 and the outer surface 134. The total number of K-wire holes 138 provided in the head portion 130 is not intended to limit this disclosure.

A relief 168 (see Figure 10) may be formed in head portion 130 on the bone contacting surface 32 of the bone plate 126. The relief 168 may be disposed slightly proximally of the elliptical hole 166 and the first opening 154. The relief 168 may be sized and shaped to provide clearance over a soft tissue anatomy associated with the distal section 24 of the humerus 16.

A plurality of scalloped portions 170 (see Figures 10 and 11) may be formed in the shaft portion 128 on the bone contacting surface 132 of the bone plate 126. The scalloped portions 170 are configured to minimize bone contact at certain locations of the bone plate 126, thereby providing a more constant stiffness across the bone plate 126 when implanted.

The longitudinal axis A may bisect the distal straight section 182 of the shaft portion 128 into two equal sections such that the distal straight section 182 is substantially symmetrical about the longitudinal axis A. The proximal tip section 180 of the shaft portion 128 and the head portion 130 may each be asymmetrically disposed about the longitudinal axis A. In an embodiment, the longitudinal axis A may intersect through an edge of the elliptical hole 166 of the head portion 130.

The bone plate 126 may be made from any biocompatible material or combination of biocompatible materials. Exemplary materials that may be suitable for manufacturing the bone plate 126 include, but are not limited to, titanium, titanium alloys, stainless steel, thermoplastic materials, etc.

The various openings 136, 158, 160, and 162 of the bone plate 126 may be configured to receive fixation screws of any size (e.g., 3.0 mm, 3.4 mm, etc.), and some of the openings may be sized differently than the other openings. Thus, the specific sizes of the openings of the bone plate 26 are not intended to limit this disclosure.

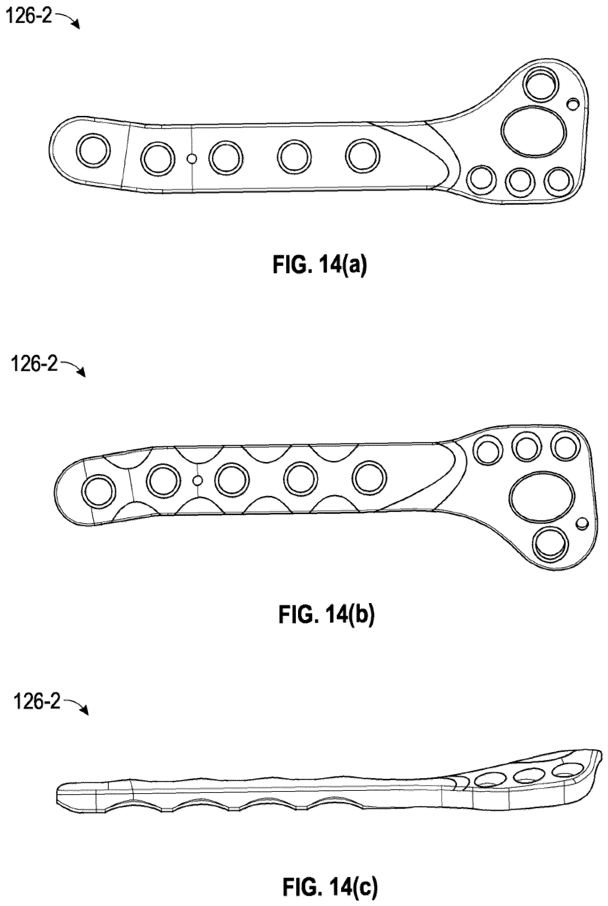

In the above embodiment, the bone plate 126 is shown and described as having a right orientation. However, the bone plate 126 could also be provided in a left orientation, with such a bone plate being the mirror image of the right orientated bone plates described above. Figures 14(a), 14(b), and 14(c) illustrate an exemplary bone plate 126-2 having a left orientation.

The bone plates of this disclosure provide a more anatomic, easier to use, and more stable bone plate for repairing distal humerus fractures of animals compared to prior plate designs. The proposed bone plates may be utilized for all distal humerus fracture patterns and include various design features for specifically accommodating the contour and native tissue anatomy of the distal humerus.

Although the different non-limiting embodiments are illustrated as having specific components or steps, the embodiments of this disclosure are not limited to those particular combinations. It is possible to use some of the components or features from any of the non-limiting embodiments in combination with features or components from any of the other non-limiting embodiments.

It should be understood that like reference numerals identify corresponding or similar elements throughout the several drawings. It should further be understood that although a particular component arrangement is disclosed and illustrated in these exemplary embodiments, other arrangements could also benefit from the teachings of this disclosure.

The present invention is defined by the appended claims.

## Claims

1. A bone plate (26, 126) designed for veterinary use, comprising:
a shaft portion (28, 128);
a head portion (30, 130);
a bone contacting surface (32, 132);
an outer surface (34, 134) opposed to the bone contacting surface (32, 132);
**characterized in that**
an elliptical hole (66, 166) is formed through the head portion (30, 130) and
is configured for accommodating at least a portion of a condyle of a distal humerus of an animal.

2. The bone plate (26, 126) as recited in claim 1, wherein the distal humerus is a canine distal humerus, and further wherein the bone contacting surface (32, 132) includes a curvature configured to match a contour of the canine distal humerus.

3. The bone plate (26, 126) as recited in claim 1 or 2, comprising a relief formed in the bone contacting surface (32, 132) of the head portion (30, 130).

4. The bone plate (26, 126) as recited in any preceding claim, wherein the shaft portion (28, 128) includes a first length that is greater than a second length of the head portion (30, 130), and the head portion (30, 130) includes a first width that is greater than a second width of the shaft portion (28, 128).

5. The bone plate (26, 126) as recited in any preceding claim, comprising a plurality of openings (36) and at least one K-wire hole (38, 138) formed through the shaft portion (28, 128), and optionally comprising a plurality of openings (36) and at least one K-wire hole (38, 138) formed through the head portion (30, 130).

6. The bone plate (26, 126) as recited in any preceding claim, wherein the head portion (30, 130) is bounded by a peripheral edge (40, 140) that connects the head portion (30, 130) to a first side and a second side of the shaft portion (28, 128).

7. The bone plate (26, 126) as recited in claim 6, wherein the peripheral edge (40, 140) includes a curved expanded section (42, 142) that connects to the first side of the shaft portion (28, 128) and an outcropped section (184) that connects to the second side of the shaft portion (28, 128).

8. The bone plate (26, 126) as recited in claim 7, comprising a first opening (54, 154) proximate the curved expanded section (42, 142) and a second opening (58, 158) proximate the outcropped section, and optionally wherein the first opening (54, 154) includes a larger diameter than the second opening (58, 158).

9. The bone plate (26, 126) as recited in claim 6, wherein the peripheral edge (40, 140) includes a curved expanded section (42, 142) that connects to the first side of the shaft portion (28, 128) and a straight edge that connects to the second side of the shaft portion (28, 128).

10. The bone plate (26, 126) as recited in claim 9, comprising a first opening (54, 154) proximate the curved expanded section (42, 142) and a second opening (58, 158), a third opening (60, 160), and a fourth opening (62, 162) proximate the straight edge, and optionally wherein the elliptical hole (66, 166) is positioned axially between the first opening (54, 154) and the second opening (58, 158) and the third opening (60, 160).

11. The bone plate (26, 126) as recited in any preceding claim, wherein the head portion (30, 130) is angled at an inclined angle relative to the shaft portion (28, 128).

12. A bone plate (26, 126) according to claim 1,
**characterized in that**
the shaft portion (28, 128) includes a first width;
the head portion (30, 130) includes a second width that is greater than the first width;
the bone contacting surface (32, 132) is configured to conform to a contour of a distal humerus of a non-human animal;
and
the head portion (30, 130) is bounded by a peripheral edge (40, 140) that connects the head portion (30, 130) to a first side and a second side of the shaft portion (28, 128),
wherein the peripheral edge (40, 140) includes a curved expanded section (42, 142) that connects to the first side of the shaft portion (28, 128) and an outcropped section (184) that connects to the second side of the shaft portion (28, 128).

13. The bone plate (26, 126) as recited in claim 12, wherein the distal humerus is a canine distal humerus.

14. The bone plate (26, 126) as recited in claim 13, wherein the elliptical hole (66, 166) is positioned axially between a first opening (54, 154) located proximate the curved expanded section (42, 142) and a third opening (60, 160) and a fourth opening (62, 162) formed proximate a straight section (48, 148) that connects to the outcropped section (184), and optionally comprising a fourth opening (62, 162) located proximate the outcropped section (184).

15. The bone plate (26, 126) as recited in any of claims 12 to 14, wherein the shaft portion (28, 128) includes a proximal tip section (180) that is angled relative to a distal straight section (48, 148) of the shaft portion (28, 128).

## Patentansprüche

1. Knochenplatte (26, 126), die für die veterinärmedizinische Verwendung konzipiert ist, umfassend:
einen Schaftabschnitt (28, 128);
einen Kopfabschnitt (30, 130);
eine Knochenkontaktfläche (32, 132);
eine Außenfläche (34, 134), die der Knochenkontaktfläche (32, 132) gegenüberliegt;
**dadurch gekennzeichnet, dass**
ein elliptisches Loch (66, 166) durch den Kopfabschnitt (30, 130) hindurch ausgebildet ist und zur Aufnahme mindestens eines Abschnitts eines Kondylus eines distalen Humerus eines Tieres ausgestaltet ist.

2. Knochenplatte (26, 126) nach Anspruch 1, wobei der distale Humerus ein distaler Humerus eines Hundes ist, und wobei die Knochenkontaktfläche (32, 132) ferner eine Krümmung aufweist, die so ausgestaltet ist, dass sie einer Kontur des distalen Humerus eines Hundes entspricht.

3. Knochenplatte (26, 126) nach Anspruch 1 oder 2, umfassend eine Aussparung, die in der Knochenkontaktfläche (32, 132) des Kopfabschnitts (30, 130) ausgebildet ist.

4. Knochenplatte (26, 126) nach einem der vorhergehenden Ansprüche, wobei der Schaftabschnitt (28, 128) eine erste Länge aufweist, die größer als eine zweite Länge des Kopfabschnitts (30, 130) ist, und der Kopfabschnitt (30, 130) eine erste Breite aufweist, die größer als eine zweite Breite des Schaftabschnitts (28, 128) ist.

5. Knochenplatte (26, 126) nach einem der vorhergehenden Ansprüche, umfassend eine Vielzahl von Öffnungen (36) und mindestens ein Kirschnerdrahtloch (38, 138), das durch den Schaftabschnitt (28, 128) hindurch ausgebildet ist, und optional umfassend eine Vielzahl von Öffnungen (36) und mindestens ein Kirschnerdrahtloch (38, 138), das durch den Kopfabschnitt (30, 130) hindurch ausgebildet ist.

6. Knochenplatte (26, 126) nach einem der vorhergehenden Ansprüche, wobei der Kopfabschnitt (30, 130) durch eine Umfangskante (40, 140) begrenzt ist, die den Kopfabschnitt (30, 130) mit einer ersten Seite und einer zweiten Seite des Schaftabschnitts (28, 128) verbindet.

7. Knochenplatte (26, 126) nach Anspruch 6, wobei die Umfangskante (40, 140) einen gekrümmten erweiterten Teilabschnitt (42, 142), der mit der ersten Seite des Schaftabschnitts (28, 128) verbunden ist, und einen vorstehenden Teilabschnitt (184), der mit der zweiten Seite des Schaftabschnitts (28, 128) verbunden ist, aufweist.

8. Knochenplatte (26, 126) nach Anspruch 7, umfassend eine erste Öffnung (54, 154) in der Nähe des gekrümmten erweiterten Teilabschnitts (42, 142) und eine zweite Öffnung (58, 158) in der Nähe des vorstehenden Teilabschnitts, und wobei optional die erste Öffnung (54, 154) einen größeren Durchmesser als die zweite Öffnung (58, 158) aufweist.

9. Knochenplatte (26, 126) nach Anspruch 6, wobei die Umfangskante (40, 140) einen gekrümmten erweiterten Teilabschnitt (42, 142), der mit der ersten Seite des Schaftabschnitts (28, 128) verbunden ist, und eine gerade Kante, die mit der zweiten Seite des Schaftabschnitts (28, 128) verbunden ist, aufweist.

10. Knochenplatte (26, 126) nach Anspruch 9, umfassend eine erste Öffnung (54, 154) in der Nähe des gekrümmten erweiterten Teilabschnitts (42, 142) und eine zweite Öffnung (58, 158), eine dritte Öffnung (60, 160) und eine vierte Öffnung (62, 162) in der Nähe der geraden Kante, und wobei optional das elliptische Loch (66, 166) axial zwischen der ersten Öffnung (54, 154) und der zweiten Öffnung (58, 158) und der dritten Öffnung (60, 160) angeordnet ist.

11. Knochenplatte (26, 126) nach einem der vorhergehenden Ansprüche, wobei der Kopfabschnitt (30, 130) in einem geneigten Winkel relativ zu dem Schaftabschnitt (28, 128) abgewinkelt ist.

12. Knochenplatte (26, 126) nach Anspruch 1,
**dadurch gekennzeichnet, dass**
der Schaftabschnitt (28, 128) eine erste Breite aufweist;
der Kopfabschnitt (30, 130) eine zweite Breite aufweist, die größer als die erste Breite ist;
die Knochenkontaktfläche (32, 132) so ausgestaltet ist, dass sie einer Kontur eines distalen Humerus eines nicht menschlichen Tieres entspricht;
und
der Kopfabschnitt (30, 130) durch eine Umfangskante (40, 140) begrenzt ist, die den Kopfabschnitt (30, 130) mit einer ersten Seite und einer zweiten Seite des Schaftabschnitts (28, 128) verbindet,
wobei die Umfangskante (40, 140) einen gekrümmten erweiterten Teilabschnitt (42, 142), der mit der ersten Seite des Schaftabschnitts (28, 128) verbunden ist, und einen vorstehenden Teilabschnitt (184), der mit der zweiten Seite des Schaftabschnitts (28, 128) verbunden ist, aufweist.

13. Knochenplatte (26, 126) nach Anspruch 12, wobei der distale Humerus ein distaler Humerus eines Hundes ist.

14. Knochenplatte (26, 126) nach Anspruch 13, wobei das elliptische Loch (66, 166) axial zwischen einer ersten Öffnung (54, 154), die sich in der Nähe des gekrümmten erweiterten Teilabschnitts (42, 142) befindet, und einer dritten Öffnung (60, 160) und einer vierten Öffnung (62, 162), die in der Nähe eines geraden Teilabschnitts (48, 148) ausgebildet sind, der mit dem vorstehenden Teilabschnitt (184) verbunden ist, angeordnet ist, und optional umfassend eine vierte Öffnung (62, 162), die in der Nähe des vorstehenden Teilabschnitts (184) angeordnet ist.

15. Knochenplatte (26, 126) nach einem der Ansprüche 12 bis 14, wobei der Schaftabschnitt (28, 128) einen proximalen Spitzenabschnitt (180) aufweist, der relativ zu einem distalen geraden Teilabschnitt (48, 148) des Schaftabschnitts (28, 128) abgewinkelt ist.

## Revendications

1. Plaque osseuse (26, 126) conçue pour un usage vétérinaire, comprenant :
une partie tige (28, 128) ;
une partie tête (30, 130) ;
une surface de contact avec l'os (32, 132) ;
une surface externe (34, 134) opposée à la surface de contact avec l'os (32, 132) ;
**caractérisée en ce que**
un trou elliptique (66, 166) est formé à travers la partie tête (30, 130) et est configuré pour loger au moins une partie d'un condyle d'un humérus distal d'un animal.

2. Plaque osseuse (26, 126) selon la revendication 1, dans laquelle l'humérus distal est un humérus distal canin, et dans laquelle en outre la surface de contact avec l'os (32, 132) comporte une courbure configurée pour concorder avec un contour de l'humérus distal canin.

3. Plaque osseuse (26, 126) selon la revendication 1 ou 2, comprenant un relief formé dans la surface de contact avec l'os (32, 132) de la partie tête (30, 130).

4. Plaque osseuse (26, 126) selon l'une quelconque des revendications précédentes, dans laquelle la partie tige (28, 128) comporte une première longueur qui est supérieure à une deuxième longueur de la partie tête (30, 130), et la partie tête (30, 130) comporte une première largeur qui est supérieure à une deuxième largeur de la partie tige (28, 128).

5. Plaque osseuse (26, 126) selon l'une quelconque des revendications précédentes, comprenant une pluralité d'ouvertures (36) et au moins un trou de broche K (38, 138) formé à travers la partie tige (28, 128), et comprenant éventuellement une pluralité d'ouvertures (36) et au moins un trou de broche K (38, 138) formé à travers la partie tête (30, 130).

6. Plaque osseuse (26, 126) selon l'une quelconque des revendications précédentes, dans laquelle la partie tête (30, 130) est délimitée par un bord périphérique (40, 140) qui relie la partie tête (30, 130) à un premier côté et un deuxième côté de la partie tige (28, 128).

7. Plaque osseuse (26, 126) selon la revendication 6, dans laquelle le bord périphérique (40, 140) comporte une section agrandie courbée (42, 142) qui se relie au premier côté de la partie tige (28, 128) et une section affleurante (184) qui se relie au deuxième côté de la partie tige (28, 128).

8. Plaque osseuse (26, 126) selon la revendication 7, comprenant une première ouverture (54, 154) à proximité de la section agrandie courbée (42, 142) et une deuxième ouverture (58, 158) à proximité de la section affleurante, et éventuellement dans laquelle la première ouverture (54, 154) comporte un diamètre plus grand que la deuxième ouverture (58, 158).

9. Plaque osseuse (26, 126) selon la revendication 6, dans laquelle le bord périphérique (40, 140) comporte une section agrandie courbée (42, 142) qui se relie au premier côté de la partie tige (28, 128) et un bord droit qui se relie au deuxième côté de la partie tige (28, 128).

10. Plaque osseuse (26, 126) selon la revendication 9, comprenant une première ouverture (54, 154) à proximité de la section agrandie courbée (42, 142) et une deuxième ouverture (58, 158), une troisième ouverture (60, 160) et une quatrième ouverture (62, 162) à proximité du bord droit, et dans laquelle éventuellement le trou elliptique (66, 166) est positionné axialement entre la première ouverture (54, 154) et la deuxième ouverture (58, 158) et la troisième ouverture (60, 160).

11. Plaque osseuse (26, 126) selon l'une quelconque des revendications précédentes, dans laquelle la partie tête (30, 130) est inclinée selon un angle incliné par rapport à la partie tige (28, 128).

12. Plaque osseuse (26, 126) selon la revendication 1, **caractérisée en ce que**
la partie tige (28, 128) comporte une première largeur ;
la partie tête (30, 130) comporte une deuxième largeur qui est supérieure à la première largeur ;
la surface de contact avec l'os (32, 132) est configurée pour épouser un contour d'un humérus distal d'un animal non humain ;
et
la partie tête (30, 130) est délimitée par un bord périphérique (40, 140) qui relie la partie tête (30, 130) à un premier côté et un deuxième côté de la partie tige (28, 128),
dans laquelle le bord périphérique (40, 140) comporte une section agrandie courbée (42, 142) qui se relie au premier côté de la partie tige (28, 128) et une section affleurante (184) qui se relie au deuxième côté de la partie tige (28, 128).

13. Plaque osseuse (26, 126) selon la revendication 12, dans laquelle l'humérus distal est un humérus distal canin.

14. Plaque osseuse (26, 126) selon la revendication 13, dans laquelle le trou elliptique (66, 166) est positionné axialement entre une première ouverture (54, 154) située à proximité de la section agrandie courbée (42, 142) et une troisième ouverture (60, 160) et une quatrième ouverture (62, 162) formées à proximité d'une section droite (48, 148) qui se relie à la section affleurante (184), et comprenant éventuellement une quatrième ouverture (62, 162) située à proximité de la section affleurante (184).

15. Plaque osseuse (26, 126) selon l'une quelconque des revendications 12 à 14, dans laquelle la partie tige (28, 128) comporte une section de pointe (180) proximale qui est inclinée par rapport à une section droite (48, 148) distale de la partie tige (28, 128).
